(19) Europäisches Patentamt
European Patent Office
Office européen des brevets

(11) Publication number: **0 356 143 B1**

(12) # EUROPEAN PATENT SPECIFICATION

(45) Date of publication of patent specification: **12.01.94**

(51) Int. Cl.$^5$: **A61K 31/44**, A61K 47/00, A61K 9/00

(21) Application number: **89308352.7**

(22) Date of filing: **17.08.89**

(54) **Injectable solutions.**

(30) Priority: **18.08.88 JP 205100/88**

(43) Date of publication of application:
**28.02.90 Bulletin 90/09**

(45) Publication of the grant of the patent:
**12.01.94 Bulletin 94/02**

(84) Designated Contracting States:
**AT BE CH DE ES FR GB GR IT LI LU NL SE**

(56) References cited:
**EP-A- 0 124 459**
**EP-A- 0 237 200**
**DE-A- 1 804 450**

**J. PHARM. PHARMACOL., vol. 36, 1984, pages 470-472, J. Pharm. Pharmacol.; L.K. WEBSTER et al.: "Effect of omeprazole and polyethylene glycol-400 on antipyrine elimination by the isolated perfused rat liver"**

**Einführung in die Verfahrenstechnik der Arzneiformung, Gstirner F., 1973, Wissensch. Verlagsgesellschaft MBH (Stuttgart) pp. 255, 271-275**

(73) Proprietor: **Takeda Chemical Industries, Ltd.**
**1-1, Doshomachi 4-chome**
**Chuo-ku, OSAKA(JP)**

(72) Inventor: **Mikura, Yasushi**
**30-11, Senriyamahigashi 4-chome**
**Suita Osaka 565(JP)**
Inventor: **Nagai, Akihiro**
**7-18, Higashitokiwadai 7-chome**
**Toyono-cho**
**Toyono-gun Osaka 563-01(JP)**
Inventor: **Shimizu, Hisayoshi**
**20-9, Teratani-cho**
**Takatsuki**
**Osaka 569(JP)**

(74) Representative: **Lewin, John Harvey et al**
**Elkington and Fife**
**Prospect House**
**8 Pembroke Road**
**Sevenoaks, Kent TN13 1XR (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

This invention relates to injectable solutions containing pyridine derivatives (hereinafter referred to, in some instances, as "Compound (I)") of the below-described formula which are useful as an anti-ulcer agent:

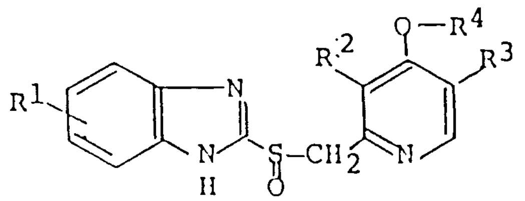

wherein $R^1$ represents hydrogen, methoxy or trifluoromethyl; $R^2$ and $R^3$, each being the same as, or different from, the other, represent hydrogen or methyl; and $R^4$ represents fluorinated $C_2$ to $C_5$ lower alkyl.

There have been known injectable solutions (refer to EP-A-124495), which are obtained by dissolving omeprazole sodium salt having anti-ulcer activity in sterilized water, followed by filtration and lyophilization to give a lyophilized material, which is then dissolved in a sterile-filtered mixed solution of polyethylene glycol 400 for injection, sodium dihydrogenphosphate and sterilized water.

In developing injectable solutions of Compound (I), there are encountered some problems being attributable to its characteristic properties.

Among the species of Compound (I), for example, 2-(3-methyl-4-trifluoroethoxy-2-pyridyl)-methylsulfinylbenzimidazole (hereinafter referred to briefly as "Compound (I-1)) shows a certain degree of solubility in water but only in the strongly alkaline region of pH 11 or more, with an extremely low solubility in the pH range below pH 11 (33 mg/ml at pH 13, 1 mg/ml at pH 11 and 0.06 to 0.0 1 mg/ml at pH 9 to 3). Referring to the stability in an aqueous solution, Compound (I-1) is satisfactorily stable in an alkaline solution, but becomes less stable as the pH of the aqueous solution is decreased to the neutral to acid range, while the resulting solution turns in appearance dark purple in a short period of time.

Thus, Compound (I), because of its characteristic properties as described above, has been difficult to process into the dosage form of injectable solution at the physiologically allowable pH range using water alone as a solvent and without employing sophisticated processing techniques, because injectable solutions favorably exhibit a pH value not being far from neutrality in terms of hemolysis, pain or local irritation.

Gstirner, F. Einfuhrung in Der Verfahrenstechnik Der Arzneiformung, 1973, Wissenschaftliche Verlagsgesellschaft mbH (Stuttgart), pp. 255, 271-275 discloses that alcohols are used in injectable solutions as solvents for water-insoluble or poor water-soluble substances. Webster, L.K.: J. Pharm. Pharmacol. Vol. 36, 1984, pp. 470-472 discloses an injectable solution of omeprazole dissolved 100% PEG-400 or in 10% PEG-400 in sodium hydrogencarbonate buffer.

In view of the circumstances described above, the present inventors, after extensive investigation, found that Compound (II) is very soluble in solvents, such as ethanol, propylene glycol and polyethylene glycol, its stability in said solvents being excellent, and that the powder produced by lyophilizing an alkaline solution of Compound (I) does not tend to discolor with the time and is extremely soluble in the above solvents, and the findings, coupled with further research, have culminated into the present invention.

Thus, the present invention provides an injectable solution which comprises a solution wherein a freeze-dried material of an alkaline aqueous solution of a compound represented by the formula:

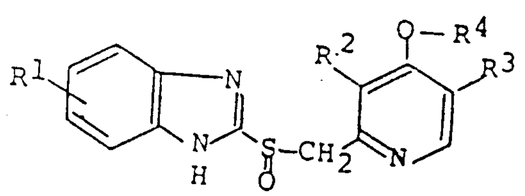

wherein $R^1$ represents hydrogen, methoxy or trifluoromethyl group; $R^2$ and $R^3$, being the same as or different from each other, represent hydrogen or methyl group; and $R^4$ represents a fluorinated $C_2$ to $C_5$ lower alkyl group, has been dissolved with a mixed solution of (a) an acidic substance, and (b) polyethylene glycol, and wherein the injectable solution also contains N-methylglucamine.

In the above-described formula, examples of the fluorinated $C_2$ to $C_5$ lower alkyl represented by $R^4$ include 2,2,2-trifluoroethyl, 2,2,3,3,3-pentafluoropropyl, 2,2,3,3-tetrafluoropropyl, 1,1,1-trifluoromethyl-2,2,2-trifluoroethyl, 2,2,3,3,4,4,4-heptafluorobutyl and 2,2,3,3,4,4,5,5-octafluoropentyl. In the above formula, preferably, $R^1$ and $R^3$ are hydrogen, and $R^2$ is methyl, with $R^4$ being 2,2,2-trifluoroethyl.

It is to be noted that the above-mentioned Compound (I) is a known compound decribed in EP-A-174726.

As the polyethylene glycol, there can be used polyethylene glycols having varied average molecular weights, and favorably usable are those having preferably an average molecular weight of 50 to 2000, more preferably an average molecular weight of 100 to 600.

The injectable solutions according to the present invention can be obtained by dissolving Compound (I) in the form of amorphous powder or crystalline powder in the above-mentioned solvent, but it is preferable to dissolve a lyophilized material of an alkaline aqueous solution of Compound (I) in a mixed solution composed of an acidic substance and the above-mentioned solvent.

As the alkaline aqueous solution of Compound (I), there may be mentioned, for example, an aqueous solution produced by dissolving in water Compound (I) in conjunction with a strongly basic substance such as sodium hydroxide, potassium hydroxide, sodium carbonate and arginine, and adjusting the resulting solution to a pH of not less than 11, preferably not less than 12.

Among the strongly basic substances, sodium hydroxide is particularly preferred.

The concentration of Compound (I) in said alkaline aqueous solution may be of any concentration only if it permits lyophilization, and is preferably 2 to 30 mg/ml, more preferably 5 to 30 mg/ml.

In order to provide the resulting lyophylized material with improved solid-forming property, it is preferred to add to the alkaline aqueous solution of compound (I) sugars, such as mannitol, inositol, maltose, sucrose, and lactose, neutral amino acids, such as glycine, alanine, proline, valine and methionine and inorganic salts, such as sodium succinate. These additives can be employed usually in an amount of 0.2 to 5 mg, preferably 0.5 to 3 mg, per mg of Compound (I).

Among these solid-forming agents, mannitol and glycine are preferable, with mannitol being particularly preferred.

The lyophilized material can be produced by lyophylizing the alkaline aqueous solution of Compound (I) by use of a method known per se, and in general, lyophilization is preferably carried out by means of a method which comprises freezing the solution at a temperature of below -25°C and drying the freezed material by warming a tray up to 25°C or 40°C at a rate of 5 to 20°C/hr while keeping the degree of vacuum in a dryer at not more than about 13.3 Pa (0.1 Torr).

The lyophilized material obtained in this manner produces the appearance of white lump form or powder form, and hardly varies in appearance with the time, thereby offering the advantageous characteristic that Compound (I) can be preserved stable for a prolonged period of time.

The lyophilized material contains a strongly basic substance, and when dissolved in the above-mentioned solvent, produces a solution with a strong alkalinity. Consequently, in order to adjust the pH of the solution to a physiologically allowable range, it is preferable to incorporate into the above-mentioned solvent a specifically determined amount of an acidic substance as a pH adjusting agent. Examples of the acidic substance include inorganic acids, such as hydrochloric acid and phosphoric acid, and organic acids, such as succinic acid and tartaric acid, as well as sodium dihydrogenphosphate and glycine. Among others, hydrochloric acid or sodium dihydrogen phosphate is preferable.

Also, it is desirable to formulate an acidic substance in such a way that the pH of the injectable solutions of this invention may be adjusted finally to 7 to 11.

The solvent having the above-mentioned acidic substance admixed permits the lyophilized material containing Compound (I) to be dissolved quickly, wherein dissolution or dilution on the occasion of use is preferred.

As the injectable solution of this invention, there may be employed a liquid composition having Compound (I) in conjunction with a strongly basic substance like the previously mentioned ones dissolved in the above-described solvent, which is to be diluted with a solvent being admixed with the above-mentioned acidic substance on the occasion of use to adjust its pH to the physiologically allowable range (ca. 7 to 11).

The thus prepared injectable solution of this invention desirably has a concentration of Compound (I) of 0.1 to 20 mg/ml, particularly 2 to 10 mg/ml.

In addition, it is preferable to incorporate the injectable solution according to the present invention with additives, including buffer solutions for the stablization of its pH, such as arginine, glycine, sodium dihydrogenphosphate, bisodium hydrogenphosphate, isotonizing agents for the adjustment of its osmotic pressure, such as sodium chloride, stabilizers, such as sodium hydrogensulfite, pain-relieving agents, such

as glucose, sorbitol, mannitol, benzyl alcohol, mepivacaine hydrochloride and Xylocaine hydrochloride, and preservatives, such as methyl p-oxybenzoate, propyl p-oxybenzoate, thymelosal and chlorobutanol, as the case may be. These substances can be added usually in an amount of 0.2 to 10 mg, preferably 0.5 to 5 mg, per mg of Compound (I).

In order to enhance the solubility of Compound (I), sodium chloride, magnesium chloride and potassium chloride can be added to the formulation. The amount of these salts in the formulation is usually 1 to 30 mg, preferably 3 to 18 mg, per mg of Compound (I).

The injectable solution of this invention is preferably prepared normally by means of the sterile preparation method known per se.

Preferably, the injectable solution according to the present invention is normally administered intravenously, and its dosage amount is desirably selected in such a way that it may be 5 to 100 mg of Compound (I) daily for male adults, preferably 10 to 50 mg.

In the injectable solution of this invention, the use as a solvent of polyethylene glycol renders Compound (I) slightly soluble in water to solubilize and to be provided with the desired degree of stability. Consequently, it becomes possible to have Compound (I) demonstrate its excellent anti-ulcer activity in an adequate manner.

Below described are the examples to illustrate this invention specifically.

Example 1 (Reference)

Compound (I-1) was dissolved in ethanol and propylene glycol to the concentration of 2 mg/ml, respectively, and the resulting solutions were sterile filtered and filled in 5-ml portions into ampoules of a 5-ml capacity, followed by sealing.

The test specimens as filled into the ampoules were investigated for appearance, clarity and content of Compound (I) immediately after preparation and after storage at 25 °C for 24 hours, with the results being shown in Table 1. The test specimens prepared by dissolution in ethanol and propylene glycol were observed to produce a slight change in appearance after storage at 25 °C for 24 hours, but the changes were judged to be so slight that they might in no way influence the injectable solution. The test specimens were found to be stable in terms of content of the active ingredient.

Table 1

| Stability of Compound (I-1) in the solution state: | | | | |
|---|---|---|---|---|
| Composition of test specimen (in 1 ml) | | Item of investigation | After preparation | After storage at 25 °C/24hrs. |
| Compound (I-1)<br>Ethanol | 2mg<br>1ml | Appearance<br>Clarity Content* | Colorless<br>Clear 100.0% | Yellowish<br>Clear 99.2% |
| Compound (I-1)<br>Propylene glycol | 2mg<br>1ml | Appearance<br>Clarity Content* | Colorless<br>Clear 100.0% | Light red-purple<br>Clear 103.0% |

Note,
*: As measured by use of high-performance liquid chromatography (HPLC), whereby the content determined immediately after preparation was taken as 100.0 % (the same method was employed for measurement in examples to be described in the following).
Chromatographic conditions of HPLC:
Carrier;
Nucleosil 5 $C_{18}$ (supplied by Gas-Chro Kogyo K.K. of Japan) 4.0 mm x 150 mm
Solvent;
Methanol:water:triethylamine (60:40:1, pH 7)
Detection method;
UV spectrophotometry at 285 nm

Example 2 (Reference)

Ethanol, polyethylene glycol 400, propylene glycol and water were mixed at the composition ratios as shown in Table 2 illustrated below, and 2 mg of Compound (I-1) was dissolved in 1 ml each of the resulting solvents, respectively, followed by adjustment to pH 9.0 with 5N-aqueous sodium hydroxide solution. As a control, there were prepared a suspension of 2 mg of Compound (I-1) in 1 ml of water being adjusted to pH 9.0 with 5N-aqueous sodium hydroxide solution and an aqueous solution of 2 mg of Compound (I-1) in 1 ml of 0.01N aqueous sodium hydroxide solution. These solutions were sterile-filtered by the conventional method and were filled in 5-ml portions into ampoules of a 5-ml capacity, respectively, followed by sealing (A control of the suspension was not subjected to sterile-filtration). The test specimens as filled into ampoules were investigated for each item of appearance, pH and content immediately after preparation and after storage at 25°C for 24 hours, with the results being shown in Table 2.

As may be evident from Table 2, all of the test specimens, except the controls, were observed to produce a slight change in appearance after storage at 25°C for 24 hours, but the changes were judged to be so slight that they might in no way influence the injectable solution. In addition, the test specimens were found to show slight change in content. The control specimens, when adjusted to the same pH value as other test specimens, failed to allow adequate dissolution of Compound (I-1), and were needed to be adjusted to a pH of not less than 11 in order to secure dissolution.

Table 2

| Stability of Compound (I-1) in the solution state | | | | |
|---|---|---|---|---|
| Composition of test specimen (in 1 ml) | | Item investigation | After preparation | After storage at 25°C/24 hrs. |
| Compound (I-1)<br>PEG-400*<br>Ethanol<br>Water | 2mg 0.05ml<br><br>0.35ml<br>0.6ml | Appearance<br><br>pH<br>Content | Colorless<br><br>9.0<br>100.0% | Slightly to light green-yellow<br><br>-<br>98.0% |
| Compound (I-1)<br>PEG-400 | 2mg<br>0.05ml | Appearance | Colorless | Slightly to light greentoyellow |
| Propylene glycol<br>Water | 0.35ml<br>0.6ml | pH<br>Content | 9.0<br>100.0% | -<br>94.4% |
| Compound (I-1)<br>PEG-400<br>Water | 2mg<br>0.4ml<br>0.6ml | Appearance<br>pH<br>Content | Colorless<br>9.0<br>100.0% | Slightly to light green-yellow<br>-<br>91.8% |
| Compound (I-1)<br>Ethanol<br>Propylene glycol | 2mg<br>0.5ml<br>0.5ml | Appearance<br>pH<br>Content | Colorless<br>9.0<br>100.0% | Slightly to light red-purple<br>-<br>98.0% |
| Compound (I-1) | 2mg | Appearance | White suspension | Slightly to light grey suspension |
| Water | 1ml | pH<br>Content | 9.1<br>- | -<br>- |
| Compound (I-1)<br><br>0.01N aq. sodium hydroxide sol'n. | 2mg<br><br>1ml | Appearance<br>pH<br>Content | Colorless<br>11.4<br>100.0% | Colorless<br>-<br>92.5% |

Note,
*; Polyethylene glycol having an average molecular weight of 400.

Example 3 (Reference)

Ethanol, polyethylene glycol 400, propylene glycol and water were mixed at the composition ratios as shown in Table 3 illustrated below, and 5 mg of Compound (I-1) was dissolved in 1 ml each of the resulting solvents, respectively, followed by adjustment to pH 9.0 with 5N-aqueous sodium hydroxide solution. As a control, there were prepared a suspension of 5 mg of Compound (I-1) in 1 ml of water being adjusted to pH 9.0 with 5N-aqueous sodium hydroxide solution and an aqueous solution of 5 mg of Compound (I-1) in 1 ml of 0.02N aqueous sodium hydroxide solution. These solutions were sterile-filtered by the conventional method and were filled in 5-ml portions into ampoules of a 5-ml capacity, respectively, followed by sealing (A control of the suspension was not subjected to sterile-filtration).

These test specimens as filled into ampoules were investigated for each item of appearance, pH and clarity immediately after preparation and after storage at 25 ° C for 24 hours, with the results being shown in Table 3.

As may be evident from Table 3, all of the test specimens, except the controls, were observed to produce a slight change in appearance after storage at 25 ° C for 24 hours, but the changes were judged to be so slight that they might in no way influence the injectable solution. In addition, they were observed to shown no change in clarity. The control specimens, when adjusted to the same pH value as other test specimens, failed to allow adequate dissolution of Compound (I-1), and were needed to be adjusted to a pH of not less than 11 in order to secure dissolution.

Table 3

| Stability of Compound (I-1) in the solution state | | | | |
|---|---|---|---|---|
| Composition of test specimen (in 1 ml) | | Item of investigation | After preparation | After storage at 25 ° C/24 hrs. |
| Compound (I-1)<br>Ethanol<br>Water | 5mg<br>0.6ml<br>0.4ml | Appearance<br>pH<br>Clarity | Colorless<br>9.0<br>Clear | Slightly to light green-yellow<br>-<br>Clear |
| Compound (I-1)<br>PEG-400*<br>Water | 5mg<br>0.4ml<br>0.6ml | Appearance<br>pH<br>Clarity | Colorless<br>9.0<br>Clear | Slightly to light green-yellow<br>-<br>Clear |
| Compound (I-1)<br>Ethanol<br>Propylene glycol<br>Water | 5mg<br>0.3ml<br>0.3ml<br>0.4ml | Appearance<br>pH<br>Clarity | Colorless<br>9.0<br>Clear | Slightly to light green-yellow<br>-<br>Clear |
| Compound (I01)<br>PEG-400<br>Ethanol<br>Water | 5mg<br>0.3ml<br>0.3ml<br>0.4ml | Appearance<br>pH<br>Clarity | Colorless<br>9.0<br>Clear | Slightly to light green-yellow<br>-<br>Clear |
| Compound (I-1)<br><br>Water<br>Compound (I-1) | 5mg<br><br>1ml<br>5mg | Appearance<br>pH<br>Clarity<br>Appearance | White suspension<br>8.9<br>-<br>Colorless | Slightly to light grey suspension<br>-<br>-<br>Colorless |
| 0.02N-aq. sodium hydroxide sol'n. | 1ml | pH<br><br>Clarity | 11.6<br><br>Clear | <br><br>Clear |

Example 4

A 1000 mg quantity of Compound (I-1) was dispersed in distilled water for injection, and 3 ml of 1N-aqueous sodium hydroxide solution was added to dissolve the Compound (I-1), followed by addition of water to make up the total of 50 ml and sterile filtration by the conventional method. The resulting filtrate was filled in 1 ml portions into vials of a 12 $cm^3$ capacity, followed by lyophilization by means of the

conventional technique. The lyophilized powder as contained in vials was dissolved in in Solvent A (which was composed of 50 mg of N-methylglucamine, 0.27 ml of 1N-hydrochloric acid and 2 ml of propylene glycol being admixed with ethanol to make up the total of 4 ml), Solvent B (which was composed of 50 mg of N-methylglucamine, 0.27 ml of 1N-hydrochloric acid, 1.2 ml of polyethylene glycol 400 and 1.2 ml of ethanol being admixed with distilled water for injection to make up the total of 4 ml), Solvent C (which was composed of 50 mg of N-methylglucamine, 0.27 ml of 1N-hydrochloric acid, 1.2 ml of ethanol and 1.2 ml of propylene glycol being admixed with distilled water for injection to make up the total of 4 ml) and Solvent D (which was composed of 50 mg of N-methylglucamine, 0.27 ml of 1N-hydrochloric acid and 2.5 ml of polyethylene glycol 400 being admixed with distilled water for injection to make up the total of 4 ml), respectively, to perform inspection for their solubilities as well as to conduct investigation into appearance clarity and contents immediately after dissolution and after storage at 25 °C for 24 hours.

The results are shown in Table 4. The lyophilized powder showed excellent solubilities in all of these solvents, and was able to be dissolved quickly. In addition to this, the resulting solutions were observed to produce slight changes in appearance immediately after dissolution and after storage for 24 hours, but the changes were found to be so slight that they might in no way influence the injectable solution. The solutions were found to show no change in the clarity while being observed to decrease slightly in content of Compound (I-1).

[Table 4] Stability of the lyophilized Compound (I-1) after being dissolved in vials:

| Item \ Solvent | A | B | C | D |
|---|---|---|---|---|
| Solubility | Good | Good | Good | Good |
| pH after dissolution | 8.7 | 9.0 | 9.0 | 9.0 |
| After dissolution: | | | | |
| Appearance | Colorless | Colorless | Colorless | Colorless |
| Clarity | Clear | Clear | Clear | Clear |
| Content | 100% | 100% | 100% | 100% |
| After storage at 25°C for 24 hrs.: | | | | |
| Appearance | Slightly to lightly green-yellow | | | |
| Clarity | Clear | Clear | Clear | Clear |
| Content | 97.0% | 96.5% | 96.7% | 96.1% |

Example 5

There were prepared 100 ml each of three kinds of aqueous solutions containing Compound (I-1), mannitol and sodium hydroxide at the individually different compositions per ml as shown in Table 5. The resulting aqueous solutions were sterile-filtered by the conventional method, and the filtrates were filled in 2 ml portions into vials of a 18 $cm^3$ capacity, respectively, followed by lyophilization by the conventional technique to give three kinds of lyophilized vials (V1, V2 and V3) having the formulations as shown in Table 6.

Then, there were prepared 500 ml each of five kinds of aqueous solutions containing PEG-400, sodium chloride, N-methylglucamine, sodium dihydrogenphosphate, hydrochloric acid and sodium hydroxide at the individually different compositions per each 10 ml as shown in Table 7. The aqueous solutions were sterile-filtered by the conventional method, and the resulting filtrates were filled in 10 ml portions into ampoules of a 10 ml capacity, respectively. The ampoules were sealed and sterilized by high pressure steam at 115 °C for 30 minutes to give five kinds of the solutions for dissolution (S1, S2, S3, S4 and S5) having the individual formulations as shown in Table 7.

Following the combinations as described in Table 8, then, the lyophilized vials were dissolved again in the solutions for dissolution, and the resulting drug solutions were investigated for pH and clarity immediately after preparation and after storage at 25 °C for 24 hours. As may be evident from the results shown in Table 8, the drug solutions resulting from any of the combinations were found to be stable in pH and good in clarity, whereby the lyophilized vials after being dissolved by any of the combinations exhibited good solubility.

[Table 5] Composition of the aqueous solution to be filled into vials (in 1 ml)

| Ingredient \ No. | 1 | 2 | 3 |
|---|---|---|---|
| Compound (I-1) | 15 mg | 15 mg | 15 mg |
| Mannitol | 15 mg | 50 mg | 15 mg |
| Sodium hydroxide | 2.4 mg | 2.4 mg | 4.8 mg |
| Distilled water for injection | Suitable volume to make up to the total of 1 ml. | | |

[Table 6] Composition of the lyophilized vials (per vila)

| Ingredient \ No. | V1 | V2 | V3 |
|---|---|---|---|
| Compound (I-1) | 30 mg | 30 mg | 30 mg |
| Mannitol | 30 mg | 100 mg | 30 mg |
| Sodium hydroxide | 4.8 mg | 4.8 mg | 9.6 mg |

[Table 7] Compositions after being dissolved (per 10 ml)

| Ingredient \ No. | S1 | S2 | S3 | S4 | S5 |
|---|---|---|---|---|---|
| PEG-400 | 3000 mg | 3000 mg | 3000 mg | 3000 mg | 3000 mg |
| Sodium chloride | 90 mg | 90 mg | 90 mg | 90 mg | 90 mg |
| N-methylglucamine | 10 mg | 40 mg | 40 mg | - | - |
| $NaH_2PO_4 \cdot 2H_2O$ | - | - | - | 13 mg | 50 mg |
| Hydrochloric acid | 1.1 mg | 7.7 mg | 8.4 mg | - | - |
| Sodium hydroxide | - | - | - | 2.8 mg | 11 mg |
| pH | 9.0 | 3.1 | 8.5 | 8.5 | 7.0 |

[Table 8] Stability after dissolution of lyophilized vials containing Compound (I-1)

| Combination \ Stability | | Immediately after preparation | | After storage at 25°C for 24 hrs. | |
|---|---|---|---|---|---|
| Vial | Solution for diss'n | pH | Clarity | pH | Clarity |
| V1 | S1 | 9.8 | Clear | 9.8 | Clear |
| V2 | S1 | 9.9 | Clear | 9.8 | Clear |
| V3 | S2 | 10.1 | Clear | 9.9 | Clear |
| V1 | S4 | 9.9 | Clear | 10.0 | Clear |
| V2 | S4 | 10.2 | Clear | 10.0 | Clear |
| V3 | S5 | 10.0 | Clear | 9.9 | Clear |

## Claims

**Claims for the following Contracting States : AT, BE, CH, DE, FR, GB, IT, LI, LU, NL, SE**

1. An injectable solution which comprises a solution wherein a freeze-dried material of an alkaline aqueous solution of a compound represented by the formula:

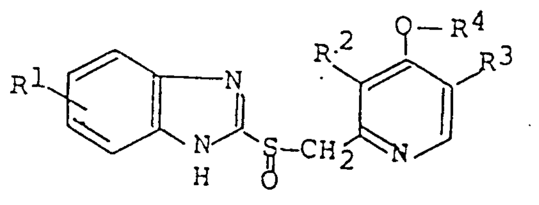

(I)

wherein $R^1$ represents hydrogen, methoxy or trifluoromethyl group; $R^2$ and $R^3$, being the same as or different from each other, represent hydrogen or methyl group; and $R^4$ represents a fluorinated $C_2$ to $C_5$ lower alkyl group, has been dissolved with a mixed solution of (a) an acidic substance, and (b) polyethylene glycol, and wherein the injectable solution also contains N-methylglucamine.

**2.** An injectable solution as claimed in claim 1, wherein the freeze-dried material is a freeze-dried material of the alkaline aqueous solution containing a saccharide besides the compound.

**3.** An injectable solution as claimed in claim 2, wherein the saccharide is mannitol.

**4.** An injectable solution as claimed in any of claims 1 to 3, wherein the acidic substance is hydrochloric acid or sodium dihydrogenphosphate.

**5.** An injectable solution as claimed in any of claims 1 to 4, wherein the polyethylene glycol is polyethylene glycol 400.

**6.** An injectable solution as claimed in any of claims 1 to 5, wherein the mixed solution additionally contains sodium chloride.

**Claims for the following Contracting States : ES, GR**

**1.** A method of preparing an injectable solution, which comprises dissolving a freeze-dried material of an alkaline aqueous solution of a compound represented by the formula:

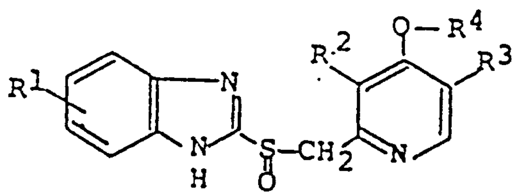

(I)

wherein $R^1$ represents hydrogen, methoxy or trifluoromethyl group; $R^2$ and $R^3$, being the same as or different from each other, represent hydrogen or methyl group; and $R^4$ represents a fluorinated $C_2$ to $C_5$ lower alkyl group, with a mixed solution of (a) an acidic substance, and (b) polyethylene glycol, and wherein the injectable solution also contains N-methylglucamine.

**2.** A method as claimed in claim 1, wherein the freeze-dried material is a freeze-dried material of the alkaline aqueous solution containing a saccharide besides the compound.

**3.** A method as claimed in claim 2, wherein the saccharide is mannitol.

**4.** A method as claimed in any of claims 1 to 3, wherein the acidic substance is hydrochloric acid or sodium dihydrogenphosphate.

**5.** A method as claimed in any of claims 1 to 4, wherein the polyethylene glycol is polyethylene glycol 400.

**6.** A method as claimed in any of claims 1 to 5, wherein the mixed solution additionally contains sodium chloride.

**Patentansprüche**
**Patentansprüche für folgende Vertragsstaaten : AT, BE, CH, DE, FR, GB, IT, LI, LU, NL, SE**

**1.** Injizierbare Lösung, die eine Lösung umfaßt, bei der ein aus einer wässrigen alkalischen Lösung einer durch die Formel

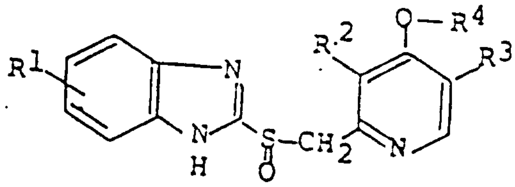

(I)

dargestellten Verbindung, worin $R^1$ ein Wasserstoffatom, eine Methoxy- oder Trifluormethylgruppe darstellt; $R^2$ und $R^3$, die untereinander gleich oder voneinander verschieden sein können, ein Wasserstoffatom oder eine Methylgruppe darstellen und $R^4$ für eine fluorierte $C_2$- bis $C_5$-Niederalkylgruppe steht, gefriergetrocknetes Material mit einer Mischlösung aus (a) einer sauren Substanz und (b) Polyäthylenglykol aufgelöst wurde, und wobei die injizierbare Lösung auch N-Methylglucamin enthält.

2. Injizierbare Lösung nach Anspruch 1, wobei das gefriergetrocknete Material ein gefriergetrocknetes Material einer wässerigen alkalischen Lösung ist, die neben der Verbindung auch ein Saccharid enthält.

3. Injizierbare Lösung nach Anspruch 2, wobei Mannit das Saccharid ist.

4. Injizierbare Lösung nach einem der Ansprüche 1 bis 3, wobei die saure Substanz Salzsäure oder Natrium-dihydrogenphosphat ist.

5. Injizierbare Lösung nach einem der Ansprüche 1 bis 4, wobei das Polyäthylenglykol Polyäthylenglykol 400 ist.

6. Injizierbare Lösung nach einem der Ansprüche 1 bis 5, wobei die Mischlösung zusätzlich Natriumchlorid enthält.

**Patentansprüche für folgende Vertragsstaaten : ES, GR**

1. Verfahren zur Herstellung einer injizierbaren Lösung, das das Auflösen eines aus einer wässrigen alkalischen Lösung einer durch die Formel

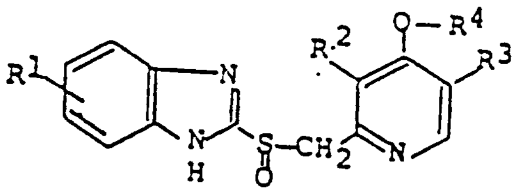

(I)

dargestellten Verbindung, worin $R^1$ ein Wasserstoffatom, eine Methoxy- oder Trifluormethylgruppe darstellt; $R^2$ und $R^3$, die untereinander gleich oder voneinander verschieden sein können, ein Wasserstoffatom oder eine Methylgruppe darstellen; $R^4$ eine fluorierte $C_2$- bis $C_5$-Nieder-Alkylgruppe darstellt, gefriergetrockneten Materials mit einer Mischlösung aus (a) einer sauren Substanz und (b) Polyäthylenglykol umfaßt, wobei die injizierbare Lösung auch N-Methylglucamin enthält.

2. Verfahren nach Anspruch 1, wobei das gefriergetrocknete Material ein gefriergetrocknetes Material einer wässerigen alkalischen Lösung ist, die neben der Verbindung auch ein Saccharid enthält.

3. Verfahren nach Anspruch 2, wobei Mannit das Saccharid ist.

4. Verfahren nach einem der Ansprüche 1 bis 3, wobei die saure Substanz Salzsäure oder Natriumdihydrogenphosphat ist.

5. Verfahren nach einem der Ansprüche 1 bis 4, wobei das Polyäthylenglykol Polyäthylenglykol 400 ist.

6. Verfahren nach einem der Ansprüche 1 bis 5, wobei die Mischlösung zusätzlich Natriumchlorid enthält.

**Revendications**

**Revendications pour les Etats contractants suivants : AT, BE, CH, DE, FR, GB, IT, LI, LU, NL, SE**

1. Solution injectable, qui comprend une solution dans laquelle le produit lyophilisé d'une solution aqueuse alcaline d'un composé répondant à la formule:

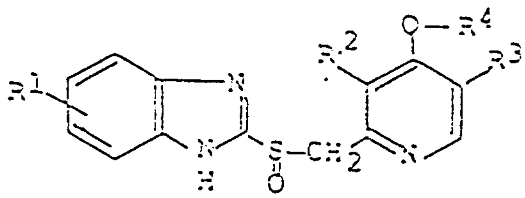

(I)

dans laquelle $R^1$ représente l'hydrogène ou un groupe méthoxy ou trifluorométhyle, $R^2$ et $R^3$, qui sont identiques ou différents l'un de l'autre, représentent l'hydrogène ou le groupe méthyle et $R^4$ représente un groupe alcoyle inférieur en $C_2$-$C_5$, fluoré,se trouve en solution dans une solution mixte (a) d'une substance acide et (b) de polyéthylène glycol et dans laquelle la solution injectable contient aussi de la N-méthylglucamine.

2. Solution injectable selon la revendication 1, dans laquelle le produit lyophilisé est le produit lyophilisé d'une solution aqueuse alcaline contenant, à part le composé, un saccharide.

3. Solution injectable selon la revendication 2, dans laquelle le saccharide est le mannitol.

4. Solution injectable selon l'une quelconque des revendications 1 à 3, dans laquelle la substance acide est l'acide chlorhydrique ou le dihydrogénophosphate de sodium.

5. Solution injectable selon l'une quelconque des revendications 1 à 4, dans laquelle le polyéthylène glycol est le polyéthylène glycol 400.

6. Solution injectable selon l'une quelconque des revendications 1 à 5, dans laquelle la solution mixte contient, en outre, du chlorure de sodium.

**Revendications pour les Etats contractants suivants : ES, GR**

1. Procédé de préparation d'une solution injectable, selon lequel on dissout le produit lyophilisé d'une solution aqueuse alcaline d'une composé répondant à la formule:

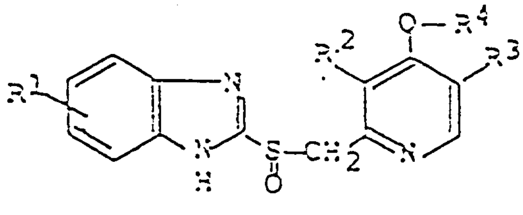

(I)

dans laquelle $R^1$ représente l'hydrogène ou un groupe méthoxy ou trifluorométhyle, $R^2$ et $R^3$, qui sont identiques ou différents l'un de l'autre, représentent l'hydrogène ou le groupe méthyle et $R^4$ représente un groupe alcoyle inférieur en $C_2$-$C_5$, fluoré,dans une solution mixte (a) d'une substance acide et (b) de polyéthylène glycol, et dans lequel la solution injectable contient aussi de la N-méthylglucamine.

2. Procédé selon la revendication 1, dans lequel le produit lyophilisé est le produit lyophilisé d'une solution aqueuse alcaline contenant, à part le composé, un saccharide.

3. Procédé selon la revendication 2, dans lequel le saccharide est le mannitol.

4. Procédé selon l'une quelconque des revendications 1 à 3, dans lequel la substance acide est l'acide chlorhydrique ou le dihydrogénophosphate de sodium.

5. Procédé selon l'une quelconque des revendications 1 à 4, dans lequel le polyéthylène glycol est le polyéthylène glycol 400.

6. Procédé selon l'une quelconque des revendications 1 à 5, dans lequel la solution mixte contient, en outre, du chlorure de sodium.